# EUROPÄISCHE PATENTSCHRIFT

(11) **EP 1 274 362 B1**
(45) Veröffentlichungstag und Bekanntmachung des Hinweises auf die Patenterteilung: **29.09.2004**
(21) Anmeldenummer: 00915079.8
(22) Anmeldetag: 17.04.2000
(51) Int. Cl.: A61B 19/02, A61L 2/26

(54) **VORRICHTUNG ZUR AUFBEWAHRUNG VON GEGENSTÄNDEN**
DEVICE FOR STORING OBJECTS
DISPOSITIF PERMETTANT DE STOCKER DES OBJETS

(43) Veröffentlichungstag der Anmeldung: 15.01.2003
(73) Patentinhaber: Mathys Medizinaltechnik AG, 2544 Bettlach (CH)
(72) Erfinder: RORATO, Gianni, CH-2540 Grenchen (CH); JANCIC, Silvin, CH-5612 Villmergen (CH); SUTER, Marcel, CH-5314 Kleindöttingen (CH)
(74) Vertreter: Lusuardi, Werther Giovanni, Dr.
(86) Internationale Anmeldenummer: PCT/CH2000/000221
(87) Internationale Veröffentlichungsnummer: WO 2001/078619

(56) Entgegenhaltungen:
- DE-U- 9 303 604
- FR-A- 2 127 815
- FR-A- 2 497 089
- US-A- 5 174 453
- US-A- 5 732 821

## Beschreibung

Die Erfindung bezieht sich auf eine Vorrichtung zur Aufbewahrung von Gegenständen, insbesondere von chirurgischen Instrumenten und/oder Implantaten gemäss dem Oberbegriff des Patentanspruchs 1.

Chirurgische Instrumente werden häufig nach Anwendungszweck geordnet und zusammen mit den entsprechenden Implantaten gelagert. Diese Lagerbehälter oder Instrumentenkasten sind üblicherweise so angefertigt, dass die Lagerbehälter zusammen mit den chirurgischen Instrumenten und Implantaten sterilisiert werden können.

Solche Instrumentenkasten bestehen meistens aus einem Behälter, mehreren in den Behälter einsetzbaren Einsatztablaren und einem verschliessbaren Deckel mit Handgriffen. Die Einsatztablare sowie der Boden des Behälter sind üblicherweise mit Hilfsmitteln zur Positionierung der Instrumente und Implantate versehen. Zur Sterilisierung sind die Seitenwände und der Boden des Behälters, der Deckel und die Einsatztablare mit durchgehenden Bohrungen ausgestattet, welche eine Verteilung des Dampfes im gesamten Instrumentenkasten zulassen.

Ein solcher Instrumentenkasten für medizinische Instrumente und Implantate ist beispielsweise aus der US 5,628,970 BASILE bekannt. Dieser bekannte Instrumentenkasten besteht aus einem Behälter, einem darin einsetzbaren Instrumententablar und einem Deckel. Am Boden des Behälters sowie am Einsatztablar sind Vertiefungen und Klemmbacken angebracht, welche eine Positionierung und Befestigung der Instrumente und Implantate ermöglichen. Alle drei Teile sind mit versenkbaren Handgriffen versehen und mittels eines am Deckel angebrachten Klemmverschlusses in zusammengefügtem Zustand verschliessbar. Der Boden des Behälters, das Einsatztablar und der Deckel sind mit Ventilationsöffnungen durchbohrt.

Nachteilig an solchen Instrumentenkasten ist, dass das Herausnehmen eines Instrumentes oder Implantates aus dem untersten Instrumententablar oder Behälterboden nach dem Abnehmen des Deckels ein aufeinanderfolgendes Herausnehmen jedes einzelnen darüberliegenden Instrumententablars erfordert, was zudem eine entsprechend grosse Ablagefläche innerhalb eines Operationsraumes erfordert. Femer sind bei diesen Ausführungsformen mit einer Aussenschale die Höhen der einzelnen Instrumententablare so zu wählen, dass die gestapelten Instrumententablare bis zum Kastendeckel reichen, um so beim Verschliessen den nötigen Anpressdruck durch den Deckel auf die Instrumententablare zu erhalten. Ansonsten fallen die Instrumententablare beim Bewegen des Kastens in der Aussenschale herum.

Ein weiterer Instrumentenkasten ist aus der FR 2 127 815 AESCULAP bekannt. Der in diesem bekannten Dokument offenbarte Verriegelungsmechanismus umfasst Klammern sowie Erhebungen an jedem der Instrumententablare, wobei jede Klammer eines unterhalb angeordneten Instrumententablars separat mit der Erhebung am oberhalb angeordneten Instrumentablar respektive am Deckel ein Eingriff gebracht werden muss.

Aus der US 5,174,453 STOEFFLER ist ebenfalls ein weiterer Instrumentkasten bekannt. In der dort offenbarten Vorrichtung muss ebenfalls jedes Instrumententablar separat mit dem nächst oberen Instrumententablar verriegelt werden. Die an den Instrumententablaren angebrachten, gegen oberen gerichteten Stifte werden durch Bohrungen am nächst oberen Instrumententablar durchgeführt und mit am jeweils oberen Instrumententablar angebrachten Schiebern einzeln verriegelt oder entriegelt.

Nachteilig an den beiden oben erwähnten Vorrichtungen ist, dass der Deckel sowie die gestapelten Instrumententablare nicht durch einen einzigen zentralen Verschlussmechanismus verriegelbar respektive lösbar sind.

Hier will die Erfindung Abhilfe schaffen. Der Erfindung liegt die Aufgabe zugrunde, einen Instrumentenkasten zu schaffen, welcher ein Aufeinanderstapeln und Verriegeln mehrerer Instrumententablare ermöglicht, ohne dass diese in einen Behälter eingefügt werden müssen. Zudem soll die Verriegelung des Deckels und der Instrumententablare synchron und zentral durch Bedienung zweier am Deckel angebrachter Handgriffe erfolgen.

Die Erfindung löst die gestellte Aufgabe mit einer Vorrichtung zur Aufbewahrung von Gegenständen, insbesondere von chirurgischen Instrumenten und/oder Implantaten, welche die Merkmale des Anspruchs 1 aufweist.

Die erfindungsgemässe Vorrichtung zur Aufbewahrung von chirurgischen Instrumenten und/oder Implantaten nach Anspruch 1 umfasst mindestens ein schalenförmiges Instrumententablar und einen auf dessen Oberseite verschliessbaren Deckel. Jedes Instrumententablar hat einen Boden mit einem vorzugsweise rechteckigen Grundriss, eine zum Boden im wesentlichen parallele Oberseite, eine Unterseite, sowie im wesentlichen senkrecht am Boden angebrachte Seitenwände. Zudem ist jedes Instrumententablar mit einem weiteren Instrumententablar stapelbar. Der Deckel besteht im wesentlichen aus einer Deckplatte mit demselben Grundriss, hat eine Deckeloberseite und eine Deckelunterseite, und umfasst mindestens einen bewegbaren Verschlussmechanismus mit gegen die Deckelunterseite gerichteten, unteren Verriegelungselementen zur Befestigung des Deckels auf der Oberseite eines Instrumententablars. Femer umfasst jedes Instrumententablar bewegbare Verriegelungsmittel, wodurch jedes Instrumententablar mit einem unterhalb angrenzenden Instrumententablar lösbar fixierbar ist. Diese Verriegelungsmittel enthalten gegen die Oberseite des Instrumententablars gerichtete, obere Verriegelungselemente und gegen die Unterseite des Instrumententablars gerichtete, untere Verriegelungselemente. Zur Fixierung des Deckels auf einem Instrumententablar sowie mehrerer Instrumententablare relativ zueinander sind die unteren Verriegelungselemente mit den oberen Verriegelungselementen und mit dem Boden eines unterhalb angrenzenden Instrumententablares lösbar in Eingriff bringbar. Dabei erfolgt der Eingriff der unteren Verriegelungselemente in die oberen Verriegelungselemente des unterhalb angrenzenden Instrumententablars senkrecht zum Grundriss und dient zur synchronen Bewegung der Verriegelungselemente aller gestapelter Instrumententablare bei einer Bewegung der Verschlussmechanismen am Deckel. Der Eingriff der unteren Verriegelungselemente mit dem Boden der unterhalb angrenzenden Instrumententablare erfolgt parallel zum Boden und dient zur lösbaren Fixation des Deckels sowie eines oder mehrerer Instrumententablare relativ zueinander und senkrecht zu deren Grundrissen.

Der Vorteil dieser Verschlussmechanismen und der so ausgeführten Verriegelungsmittel liegt darin, dass eine Verriegelung von Deckel und allen Instrumententablaren zentral durch Bedienung der am Deckel angebrachten Handgriffe erfolgen kann.

In einer Ausführungsform der erfindungsgemässen Vorrichtung weisen die Seitenwände des Instrumententablares i senkrecht zum Grundriss eine Höhe Hᵢ auf und sind von der Unterseite her bis zu einer Tiefe Tᵢ ausgekröpft, so dass auf der Innenseite der Seitenwände in der Tiefe Tᵢ eine Schulter ist, welche bei gestapelten Instrumententablaren auf dem Boden des unterhalb angrenzenden Instrumententablares aufliegt. Anstelle einer Auflage der auf der Innenseite liegenden Schulter auf dem Boden des unterhalb angrenzenden Instrumententablares, ist auch eine Ausgestaltung der ausgekröpften Seitenwände denkbar, bei welcher die Tiefe Tᵢ so bemessen ist, dass die Unterseite eines Instrumententablares auf der durch die Auskröpfung auf der Aussenseite der Seitenwände gebildeten Schulter des unterhalb angrenzenden Instrumententablares aufliegen kann. Der Vorteil dieser ausgekröpften Seitenwände ist darin zu finden, dass die unteren Verriegelungselemente nicht gegen die Unterseite nicht über die Seitenwände herausragen, so dass jedes Instrumententablar über eine ebene Unterseite verfügt. Ferner erhöhen die ausgekröpften Seitenwände die Verwindungssteifigkeit der Instrumententablare.

Die Höhe Hᵢ kann für die verschiedenen Instrumententablare variieren, während die Tiefe Tᵢ vorzugsweise standardisiert und für alle Instrumententablare dieselbe ist. Damit ist die Verriegelung zwischen den einzelnen Instrumententablaren immer gewährleistet.

Vorzugsweise ist auch der Deckel analog zu den Instrumententablaren mit ausgekröpften Seitenwänden ausgestattet.

in einer weiteren Ausführungsform der erfindungsgemässen Vorrichtung sind der Deckel und jedes Instrumententablar mit einem rechteckigen Grundriss ausgebildet und aus einem gegen Kerbschlag zähen und dampfsterilisierbaren Kunststoff, vorzugsweise PPSU (Polyphenylensulfon) gefertigt. Die Instrumententablare werden vorzugsweise durch Tiefziehen hergestellt, wodurch sich eine höhere Festigkeit erreichen lässt.

In einer anderen Ausführungsform der erfindungsgemässen Vorrichtung sind die Deckplatte des Deckels und die Böden der Instrumententablare mit Ventilationsbohrung versehen, wodurch die in der Vorrichtung gelagerten chirurgischen Instrumente und Implantate sterilisierbar sind. Die Ventilationsbohrungen weisen einen Durchmesser zwischen 3 mm und 8 mm, vorzugsweise von 6 mm auf. In dieser Ausführungsform der erfindungsgemässen Vorrichtung sind die Tiefe T_{D} an den ausgekröpften Seitenwänden am Deckel und die Tiefen Tᵢ an den ausgekröpften Seitenwänden an den Instrumententablaren so bemessen, dass beim Aufsetzen des Deckels und beim Stapeln der Instrumententablare die unteren Verriegelungselemente auf den oberen Verriegelungselementen des jeweils unterhalb angrenzenden Instrumententablars aufliegen und dadurch die Schultern an den Seitenwänden nicht auf den Böden der unterhalb angrenzenden Instrumententablare aufliegen. Der Luftspalt, welcher dadurch zwischen dem oberen und dem unteren Instrumententablar entsteht, unterstützt die Durchlüftung während dem Sterilisationsprozess.

Vorzugsweise umfasst jeder Verschlussmechanismus einen ersten stabförmigen Schieber und jedes Verriegelungsmittel einen zweiten stabförmigen Schieber. Die Schieber weisen eine parallel zur Schmalseite und parallel zum Grundriss der Deckplatte respektive des Bodens verlaufende Längsachse auf und sind parallel zu dieser Längsachse verschiebbar. Die oberen Verriegelungselemente sind als gegen die Oberseite gerichtete und senkrecht zur Längsachse an den Schiebern angebrachte Vertiefungen ausgeführt, während die unteren Verriegelungselemente als dazu korrespondierende Erhebungen an der Unterseite der Schieber angebracht sind. Der Boden jedes Instrumententablars umfasst senkrecht zum Grundriss durchgehende Verriegelungslöcher, wodurch die unteren Verriegelungselemente durchführbar sind. Die unteren Verriegelungselemente sind mit parallel zur Längsachse verlaufenden Schlitzen versehen, welche bei durch die Verriegelungslöcher eines unterhalb angrenzenden Instrumententablars geführten, unteren Verriegelungselementen mit dem Boden dieses unterhalb angrenzenden Instrumententablars durch Bewegen des Schiebers in Eingriff bringbar sind.

In einer weiteren Ausführungsform der erfindungsgemässen Vorrichtung sind die Böden der Instrumententablare auf der Oberseite mit Vertiefungen versehen, deren Form das für jede Vertiefung bestimmte chirurgische Instrument und/oder Implantat definiert. Die Vertiefungen weisen entsprechend eine bestimmte Tiefe auf, welche zusammen mit den Abmessungen Hᵢ und Tᵢ des oberen Instrumententablars zweier aufeinander zu stapelnder Instrumententablare so abgestimmt sind, dass durch die Grundflächen der Vertiefungen des oberen Instrumententablars die in die Vertiefungen des unteren Instrumententablars definiert eingelegten chirurgischen Instrumente oder Implantate senkrecht zum Boden fixierbar sind. Die Instrumententablare sind dann nur in einer bestimmten Reihenfolge stapelbar.

Die Instrumententablare weisen mit ihren Böden und den Vertiefungen eine dreidimensionale Strukturierung auf, wodurch eine zusätzliche Verwindungssteifigkeit der Instrumententablare erreicht wird.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung keine Aussenschale benötigt wird, wodurch weniger Ablagefläche im Operationsraum gebraucht wird. Ferner wird durch das Wegfallen der Aussenschale im Falle der sterilisierbaren Ausführungsform eine bessere Durchlüftung der Vorrichtung erreicht.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Darstellung einer Ausführungsform der erfindungsgemässen Vorrichtung mit einem Deckel und vier Instrumententablaren;
Fig. 2 einen Schnitt (Schnitt A-A gemäss Fig. 1) parallel zu den Schmalseiten der in Fig. 1 dargestellten Ausführungsform der erfindungsgemässen Vorrichtung;
Fig. 3 einen Schnitt (Schnitt A-A gemäss Fig. 1) parallel zu den Schmalseiten der in Fig. 1 dargestellten Ausführungsform der erfindungsgemässen Vorrichtung mit einem Deckel und einem Instrumententablar;
Fig. 4 einen Schnitt durch die Seitenwände parallel zu den Längsseiten einer Ausführungsform der erfindungsgemässen Vorrichtung; und
Fig. 5 eine perspektivische Darstellung eines Führungszapfens einer Ausführungsform der erfindungsgemässen Vorrichtung.

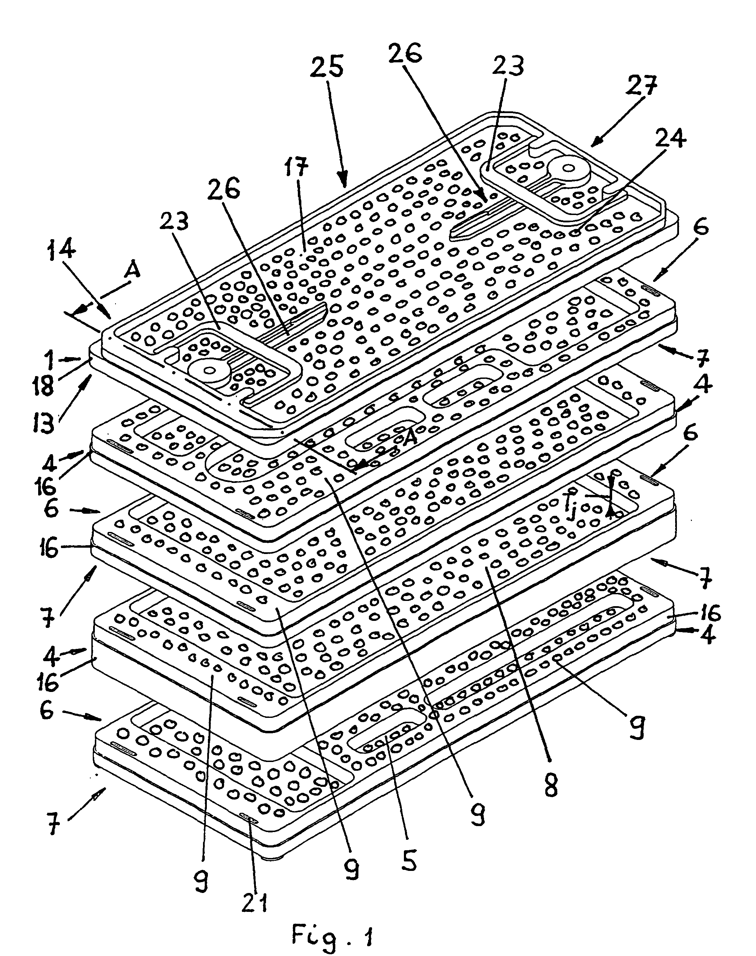

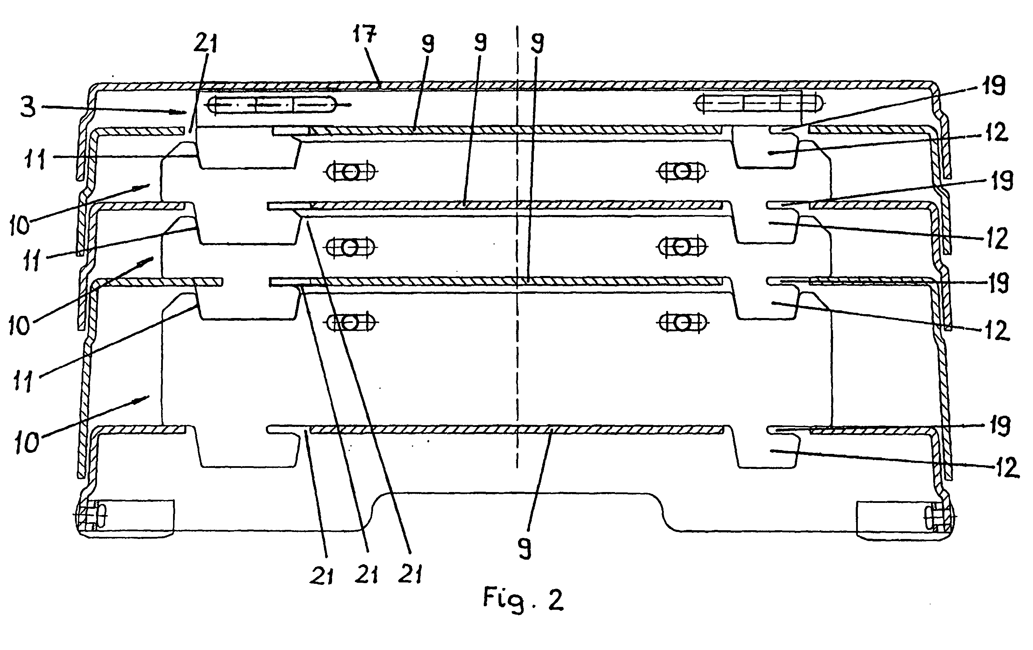

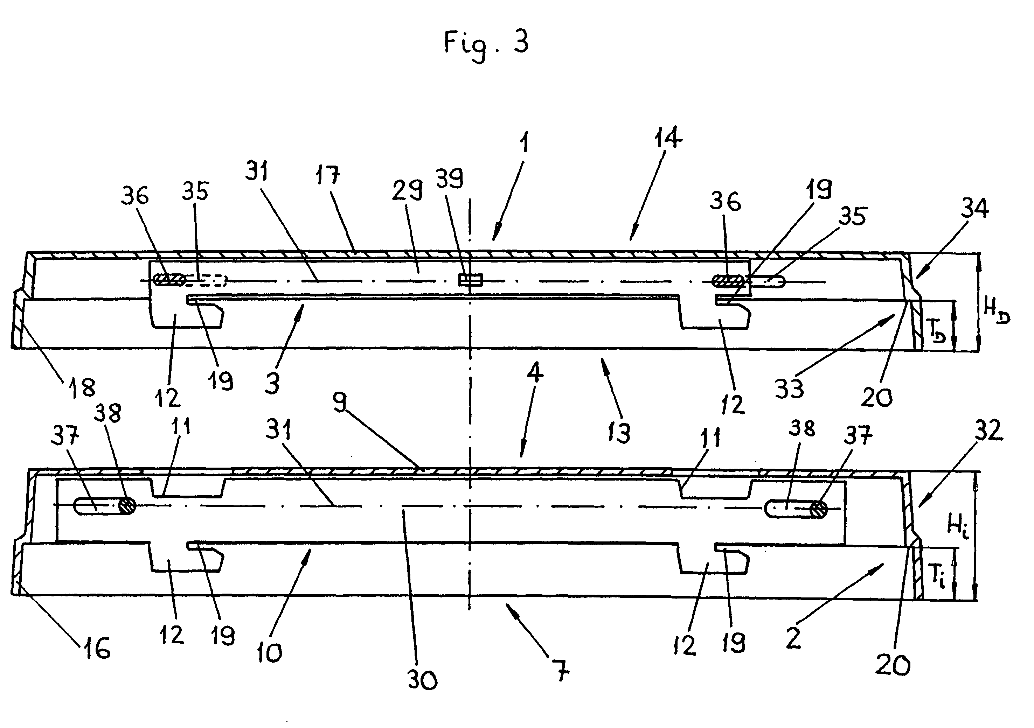

In Fig. 1 ist eine Ausführungsform der erfindungsgemässen Vorrichtung mit vier Instrumententablaren 4 und einem Deckel 1 dargestellt, wobei Deckel 1 und Instrumententablare 4 einen rechteckigen Grundriss mit zwei parallelen Schmalseiten 27 und zwei ebenfalls parallelen Längsseiten 25 aufweisen. Die Instrumententablare 4 sind schalenförmig ausgestaltet und stapelbar. Jedes Instrumententablar 4 weist einen Boden 9, eine zum Boden 9 parallele Oberseite 6, eine Unterseite 7 und auf der gesamten Peripherie des Grundrisses Seitenwände 16 auf, welche auf der Unterseite 7 der Instrumententablare 4 senkrecht auf dem Boden 9 stehen. Der Deckel 1 weist eine Deckplatte 17 mit demselben Grundriss wie die Böden 9 der Instrumententablare 4, sowie eine Deckeloberseite 14, eine Deckelunterseite 13 und auf der gesamten Peripherie des Grundrisses Seitenwände 18 auf, welche auf der Deckelunterseite 13 senkrecht auf der Deckplatte 17 stehen. Ferner sind an den Schmalseiten 27 am Deckel 1 zwei um senkrecht zur Deckplatte 17 angeordneten Drehachsen 22 schwenkbare Hangriffe 26 zur Bedienung der Verschlussmechanismen 3 (Fig. 2 und 3) sowie zwei Traggriffe 23 angebracht.

Jedes Instrumententablar 4 weist eine Anzahl n Vertiefungen 5 mit je einer Tiefe Tⱼ (j = 1 bis n) und je einer Grundfläche (Gⱼ; j = 1 bis n) 8 auf, welche zur Aufnahme von chirurgischen Instrumenten und/oder Implantaten dienen. Die durch die Grundflächen (Gⱼ; j = 1 bis n) 8 und die Tiefen Tⱼ (j = 1 bis n) bestimmten Formen der Vertiefungen 5 sind optimal auf die Aussenkonturen des jeder Vertiefung 5 zugeordneten chirurgischen Instrumentes oder Implantates abgestimmt, wodurch ein Herumfallen oder gegenseitiges Berühren der eingelegten Instrumente oder Implantate verhindert wird. Die Anordnung der Vertiefungen 5 und somit des Inhaltes auf jedem Instrumententablar 4 erfolgt vorzugsweise anhand des Operationsablaufes, wodurch die Reihenfolge der zum Einsatz kommenden Instrumente respektive Implantate bestimmt ist. Durch diese genaue Zuordnung der einzelnen Vertiefungen 5 wird die Aufbereitung des gesamten Inhaltes in der erfindungsgemässen Vorrichtung für den nächsten Einsatz im Operationsraum wesentlich erleichtert, da sehr rasch durch einen einzigen Kontrollblick feststellbar ist, ob das gesamte System komplett ist.

Die Höhe Hᵢ (Fig. 3) eines Instrumententablars (i) 4 sowie die Tiefen Tⱼ (j = 1 bis n) der n Vertiefungen 5 sind dabei so bestimmt, dass die chirurgischen Instrumente und/oder Implantate, welche in die ihnen zugeordneten Vertiefungen 5 im Instrumententablar (i) 4 eingelegt sind, durch die Grundflächen 8 des oberhalb angrenzenden Instrumententablars (i - 1) 4 senkrecht zu den Grundflächen 8 fixiert werden. Dadurch wird verhindert, dass die chirurgischen Instrumente und/oder Implantate beim Schütteln der Vorrichtung aus den Vertiefungen 5 herausfallen. Ferner sind die Seitenwände 16 von der Unterseite 7 her bis zu einer Tiefe Tᵢ ausgekröpft, so dass auf der Innenseite 2 eine Schulter 20 gebildet wird, welche beim Stapeln des Instrumententablars (i) 4 auf dem Boden 9 des unterhalb angrenzenden Instrumententablars (i + 1) 4 aufliegt.

An den Schmalseiten 27 jedes Instrumententablars 4 sind zwei Verriegelungslöcher 21 zur Aufnahme der unteren Verriegelungselemente 12 (Fig. 2 und 3) angebracht. In der sterilisierbaren Ausführungsform der erfindungsgemässen Vorrichtung sind Deckplatte 17, Böden 9 und die Grundflächen 8 der Vertiefungen 5 mit Ventilationsbohrungen 24 versehen, welche vorzugsweise einen Durchmesser von 6 mm aufweisen. Der Durchmesser der Ventilationsbohrungen 24 ist auf die Abmessungen der kleinsten Instrumente beziehungsweise Implantate abzustimmen, so dass diese nicht aus der Vorrichtung respektive nicht von einem Instrumententablar (i) 4 auf das unterhalb angrenzende Instrumententablar (i + 1) 4 fallen können. Für eine gute Ventilationswirkung während der Sterilisation ist es vorteilhaft, möglichst viele Ventilationsbohrungen 24 anzubringen.

In Fig. 2 sind ein Deckel 1 und vier Instrumententablare 4 in gestapeltem Zustand im Schnitt A-A (Fig. 1) dargestellt.

Fig. 3 zeigt den Deckel 1 und ein Instrumententablar (i) 4 in einem Schnitt (Schnitt A-A gemäss Fig. 1) parallel zu den Schmalseiten 27. Die Seitenwände 16 des Instrumententablars 4 weisen eine Innenseite 2, eine Aussenseite 32 und eine dem Instrumententablar (i) 4 zugeordnete Höhe H; auf. Die Seitenwände 18 des Deckels 1 weisen eine Innenseite 33, eine Aussenseite 34 sowie eine Höhe H_{D} auf und sind von der Deckelunterseite 13 her bis zu einer Tiefe T_{D} ausgekröpft, so dass analog zu den Instrumententablaren 4 eine Schulter 20 zur Auflage auf dem Boden 9 des unterhalb angrenzenden Instrumententablares 4 gebildet wird.

In den Fig. 2 und 3 gezeigt ist der parallel zur Deckplatte 17 bewegbare Verschlussmechanismus 3 am Deckel 1 sowie die parallel zu den Böden 9 bewegbaren Verriegelungsmittel 10 an den Instrumententablaren 4, wobei an jeder der beiden Schmalseiten 27 (Fig. 1) je ein Verschlussmechanismus 3 sowie pro Instrumententablar 4 je ein Verriegelungsmittel 10 angebracht ist. Der Verschlussmechanismus 3 umfasst zwei gegen die Deckelunterseite 13 gerichtete, untere Verriegelungselemente 12 zur Befestigung des Deckels 1 auf dem obersten Instrumententablar (i = 1) 4, während die Verriegelungselemente 10 zwei gegen die Oberseite 6 gerichtete, obere Verriegelungselemente 11 und zwei gegen die Unterseite 7 gerichtete, untere Verriegelungselemente 12 umfassen. Die unteren Verriegelungselemente 12 weisen eine zu den oberen Verriegelungselementen 11 komplementäre Form auf. Beim Stapeln mehrerer Instrumententablare 4 sowie beim Auflegen des Deckels 1 sind die unteren Verriegelungselemente 12 durch in den Instrumententablaren 4 angebrachten Verriegelungslöcher 21 führbar und senkrecht zu den Böden 9 formschlüssig mit den oberen Verriegelungselementen 11 des jeweils unterhalb angrenzenden Instrumententablars 4 in Eingriff bringbar. Durch eine Bewegung der Verschlussmechanismen 3 werden daher die Verriegelungsmittel 10 aller aufeinandergestapelter Instrumententablare 4 mitbewegt.

Die Verschlussmechanismen 3 sind als erste stabförmige Schieber 29 und die Verriegelungsmittel 10 als zweite stabförmige Schieber 30 ausgestaltet. Die Schieber 29;30 haben eine parallel zu den Schmalseiten 27 und ebenfalls parallel zum Grundriss der Deckplatte 17 respektive der Böden 9 verlaufenden Längsachse 31. Auf den Schmalseiten 27 (Fig. 1) der Seitenwände 18 sind parallel zur Längsachse 31 zwei als Langlöcher ausgestaltete Führungsbahnen 35 angebracht, worin zwei an den ersten Schiebern 29 angebrachte Führungszapfen 36 so eingefügt sind, dass die Schieber 29 parallel zur Längsachse 31 verschiebbar mit dem Deckel 1 verbunden sind. Die zweiten Schieber 30 an den Instrumententablaren 4 sind mit ebenfalls parallel zur Längsachse 31 angeordneten Langlöchern 37 versehen, in welchen an den Seitenwänden 16 befestigte Führungselemente 38 aufgenommen werden, so dass die zweiten Schieber 30 parallel zur Längsachse 31 verschiebbar mit den Instrumententablaren verbunden sind. Durch eine mit dem Handgriff 26 (Fig. 1) im Eingriff stehende Nase 39 am ersten Schieber 3 wird die Schwenkbewegung des Handgriffes 26 (Fig. 1) in eine lineare, parallel zur Längsachse 31 verlaufende Bewegung übertragen.

Die Ausgestaltung der Verriegelungsmittel 10 ist in der bevorzugten Ausführungsform der erfindungsgemässen Vorrichtung so ausgeführt, dass die unteren Verriegelungselemente 11 als keilförmige, senkrecht zur Längsachse 31 stehende Erhebungen und die oberen Verriegelungselemente 12 als ebenfalls keilförmige, senkrecht zur Längsachse 31 verlaufende Vertiefungen an den Schiebern 29;30 angebracht sind. Die als Erhebungen ausgeführten unteren Verriegelungselemente 11 weisen je einen Schlitz 19 auf. Beim Verschliessen der Verschlussmechanismen 3 durch Drehen des Handgriffes 26 werden die Schieber 29;30 parallel zur Längsachse 31 verschoben, wodurch die Schlitze 19 an den als Erhebungen ausgestalteten unteren Verriegelungselementen 11 mit dem Boden 9 des jeweils unterhalb angrenzenden Instrumententablars 4 in Eingriff gelangen und der Deckel 1 mit dem obersten Instrumententablar (i = 1) 4 sowie die unterhalb angrenzenden Instrumententablare (i = 1 bis z) relativ zueinander fixiert werden.

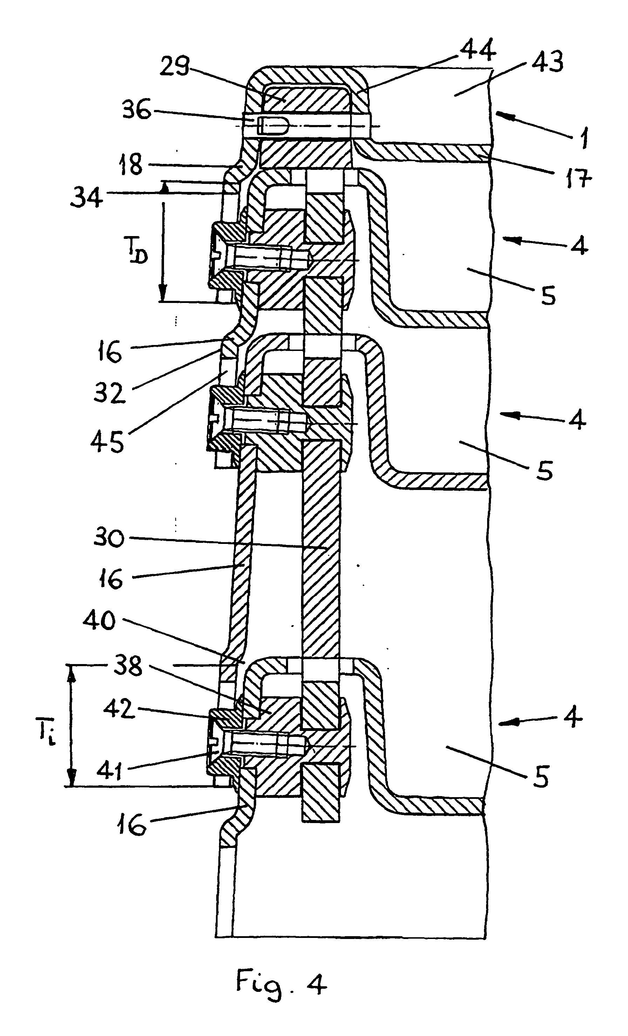

Fig. 4 zeigt einen parallel zu den Längsseiten 25 verlaufenden Schnitt durch die Seitenwände 16;18 einer aus einem Deckel 1 und drei Instrumententablaren 4 zusammengesetzten Ausführungsform der erfindungsgemässen Vorrichtung. Der Deckel 1 weist auf der Deckeloberseite 14 eine Einbuchtung 43 auf, so dass die Deckplatte 17 vertieft angeordnet ist und der Deckel 1 zusätzlich eine Innenwand 44 umfasst. Im Gegensatz zu der in Fig. 3 dargestellten Ausführungsform der erfindungsgemässen Vorrichtung sind die Führungsbahnen 35 (Fig. 3) als Langlöcher am ersten Schieber 29 angebracht, während Führungszapfen 36, welche in ovalen, an der Seitenwand 18 und der Innenwand 44 angebrachten Bohrungen im Deckel 1 gelagert sind, zur verschiebbaren Lagerung der ersten Schieber 29 am Deckel 1 dienen. Die verschiebbare Lagerung der zweiten Schieber 30 an den Instrumententablaren 4 ist so ausgestaltet, dass die zweiten Schieber 30 Langlöcher 37 (Fig. 3) aufweisen und an der Seitenwand 16 mittels eines Zwischenringes 42 und einer Schraube 41 befestigte Führungselemente 38 als in den Langlöchern 37 aufnehmbare Lagerzapfen für die zweiten Schieber 30 dienen. In der hier gezeigten Ausführungsform der erfindungsgemässen Vorrichtung sind die Tiefen T_{D} und Tᵢ so gewählt, dass beim Aussetzen des Deckels 1 sowie beim Stapeln der Instrumententablare 4 die ersten und zweiten Schieber 29;30 aufeinander aufstehen, so dass zwischen Deckel 1 und dem unterhalb angrenzenden Instrumententablar 4 sowie zwischen den einzelnen Instrumententablaren 4 ein Luftspalt 40 bleibt. Die Zwischenringe 42 stehen im wesentlichen senkrecht auf den Seitenwänden 16 und bilden somit auf der Aussenseite 32 über die Seitenwände 16 vorragende Nocken, welche beim Aufsetzen des Deckels 1 auf das oberste Instrumententablar 4 respektive beim Stapeln mehrerer Instrumententablare 4 in entsprechende, an den Seitenwänden 16;18 angebrachte und gegen die Unterseite 7 offene Öffnungen 45 in Eingriff bringbar sind. Die Zwischenringe 42 können dabei verschiedene Aussendurchmesser aufweisen, so dass durch eine komplementäre Ausgestaltung der Öffnungen 45 an den Seitenwänden 16 der Deckel 1 und die Instrumententablare 4 nur in einer bezüglich der Schmalseiten 27 respektive Längsseiten 25 definierten Ordnung gestapelt werden können und beispielsweise nicht um 180° verdreht werden können.

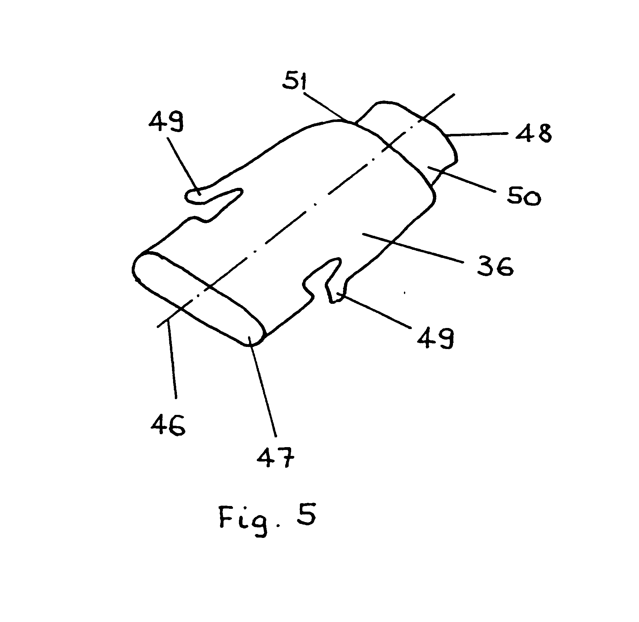

Fig. 5 zeigt einen Führungszapfen 36 der in Fig. 4 dargestellten Ausführungsform der erfindungsgemässen Vorrichtung. Der Führungszapfen 36 weist eine Zentralachse 46, ein gegen die Aussenseite 34 (Fig. 3) gerichtetes äusseres Ende 47 und ein axial entgegengesetztes inneres Ende 48 auf. Der Querschnitt senkrecht zur Zentralachse 46 ist oval. Ferner umfasst der Führungszapfen 36 am inneren Ende 48 einen Zapfen mit einem ebenfalls ovalen Zapfenquerschnitt, welcher gegenüber dem Querschnitt am äusseren Ende 47 kleinere Halbachsen aufweist, so dass ein Absatz 51 gebildet wird. Zwei Nocken 49, welche über die grosse Halbachse des Querschnittes hervorstehen, dienen zur Befestigung des Führungszapfens 36 im Deckel 1.

## Patentansprüche

1. Vorrichtung zur Aufbewahrung von Gegenständen, insbesondere von chirurgischen Instrumenten und/oder Implantaten, mit
A) mindestens einem schalenförmigen Instrumententablar (4), welches einen Boden (9), eine zum Grundriss des Bodens (9) im wesentlichen parallele Oberseite (6), eine Unterseite (7), sowie im wesentlichen senkrecht am Boden (9) angebrachte Seitenwände (16) umfasst, wobei das mindestens eine Instrumententablar (4) mit einem weiteren Instrumententablar (4) stapelbar ist; und
B) einem Deckel (1), welcher eine Deckplatte (17) mit demselben Grundriss wie der Boden (9), eine Deckeloberseite (14), eine Deckelunterseite (13) und mindestens einen bewegbaren Verschlussmechanismus (3) zur lösbaren Befestigung des Deckels (1) auf der Oberseite (6) eines Instrumententablars (4) umfasst, wobei
C) jedes Instrumententablar (4) Verriegelungsmittel (10) umfasst, durch deren Bewegung jedes Instrumententablar (4) mit einem unterhalb angrenzenden Instrumententablar (4) lösbar fixierbar ist,
**dadurch gekennzeichnet, dass**
D) die Verriegelungsmittel (10) gegen die Oberseite (6) gerichtete, obere Verriegelungselemente (11) und gegen die Unterseite (7) gerichtete, untere Verriegelungselemente (12) umfassen; und
E) der Verschlussmechanismus (3) ebenfalls gegen die Deckelunterseite (13) gerichtete, untere Verriegelungselemente (12) umfasst, wobei
F) die unteren Verriegelungselemente (12) mit den oberen Verriegelungselementen (11) eines unterhalb angrenzenden Instrumententablares (4) lösbar in Eingriff bringbar sind und die Verriegelungselemente (11;12) durch Bewegung des mindestens einen Verschlussmechanismus (3) am Deckel (1) synchron bewegbar sind.

2. Vorrichtung nach Anspruch 1, **dadurch gekennzeichnet, dass** die unteren Verriegelungselemente (12) durch Bewegung des Verschlussmechanismusses (3) parallel zum Grundriss mit dem Boden (9) eines unterhalb angrenzenden Instrumententablars (4) lösbar in Eingriff bringbar sind.

3. Vorrichtung nach Anspruch 1 oder 2, **dadurch gekennzeichnet, dass** der mindestens eine Verschlussrnechanismus (3) einen Handgriff (26) zur Bewegung des Verschlussmechanismusses (3) umfasst.

4. Vorrichtung nach einem der Ansprüche 1 bis 3, **dadurch gekennzeichnet, dass** die Seitenwände (16) eines Instrumententablars i (4) eine Höhe (Hᵢ), eine Innenseite (2) und eine Aussenseite (32) aufweisen und auf der Unterseite (7) angebracht sind, und dass die Seitenwände (16) von der Unterseite (7) her bis zu einer Tiefe (Tᵢ) ausgekröpft sind, wodurch auf der Innenseite (2) eine Schulter (20) gebildet wird.

5. Vorrichtung nach einem der Ansprüche 1 bis 4, **dadurch gekennzeichnet, dass** der Grundriss der Deckplatte (17) und jedes Bodens (9) im wesentlichen rechteckig ist und zwei parallele Längsseiten (25) und zwei ebenfalls parallele Schmalseiten (27) umfasst, wobei zwei Verschlussmechanismen (3) am Deckel (1) und zwei Verriegelungsmittel (10) an einem Instrumententablar (4) jeweils auf den Schmalseiten (27) angeordnet sind.

6. Vorrichtung nach Anspruch 4 oder 5, **dadurch gekennzeichnet, dass** die Tiefe (Tᵢ) jedes Instrumententablars i (4) so bemessen ist, dass die Schulter (20) auf dem Boden (9) eines unterhalb angrenzenden Instrumententablars i + 1 (4) zur Anlage bringbar ist.

7. Vorrichtung nach Anspruch 4 oder 5, **dadurch gekennzeichnet, dass** die Tiefe (Tᵢ) jedes Instrumententablars i (4) so bemessen ist, dass bei gestapelten Instrumententablaren (4) zwischen den Schultern (20) an den Seitenwänden (16) und den Böden (9) der jeweils unterhalb angrenzenden Instrumententablare (4) ein Luftspalt (40) verbleibt.

8. Vorrichtung nach einem der Ansprüche 1 bis 7, **dadurch gekennzeichnet, dass** die Deckplatte (17) und jeder Boden (9) mit dazu im wesentlichen senkrecht durchgehenden Ventilationsbohrungen (24) versehen sind.

9. Vorrichtung nach einem der Ansprüche 5 bis 8, **dadurch gekennzeichnet, dass** jeder Verschlussmechanismus (3) einen ersten stabförmigen Schieber (29) und jedes Verriegelungsmittel (10) einen zweiten stabförmigen Schieber (30) umfasst, wobei die Schieber (29;30) eine parallel zur Schmalseite (27) und parallel zum Grundriss der Deckplatte (17) respektive des Bodens (9) verlaufende Längsachse (31) umfassen, und dass die Schieber (29;30) parallel zu dieser Längsachse (31) verschiebbar sind.

10. Vorrichtung nach Anspruch 9, **dadurch gekennzeichnet, dass** die oberen Verriegelungselemente (11) als senkrecht zur Längsachse (31) an den Schiebern (30) angebrachte Vertiefungen und die unteren Verriegelungselemente (12) als dazu korrespondierende Erhebungen an den Schiebern (29;30) angebracht sind, der Boden (9) senkrecht zum Grundriss durchgehende Verriegelungslöcher (21) umfasst, durch welche die unteren Verriegefungselemente (12) durchführbar sind, und dass die unteren Verriegelungselemente (12) einen parallel zur Längsachse (31) verlaufenden Schlitz (9) umfassen, welcher bei durch die Verriegelungslöcher (21) eines unterhalb angrenzenden Instrumententablars (4) geführten, unteren Verriegelungselementen (12) mit dem Boden (9) dieses unterhalb angrenzenden Instrumententablars (4) durch Bewegen des Schiebers (29;30) in Eingriff bringbar ist.

11. Vorrichtung nach einem der Ansprüche 4 bis 10, **dadurch gekennzeichnet, dass** der Boden (9) eines Instrumententablares i (4) auf der Oberseite (6) n Vertiefungen (5) umfasst, deren Form das in jeder Vertiefung (5) zur Lagerung bestimmte chirurgische Instrument und/oder Implantat bestimmt, die Vertiefung j (5) eine Tiefe Tⱼ aufweist und eine Grundflächen Gⱼ (8) hat, und dass die Abmessungen Hᵢ und Tᵢ des oberen Instrumententablars (4) zweier aufeinander zu stapelnder Instrumententablare (4) und die Tiefen Tⱼ des unteren und des oberen Instrumententablars (4) so abgestimmt sind, dass durch die Grundflächen (8) des oberen Instrumententablars (4) die in der bestimmten Zuordnung in die Vertiefungen (5) des unteren Instrumententablars (4) eingelegten chirurgischen Instrumente oder Implantate senkrecht zum Boden (9) fixierbar sind.

12. Vorrichtung nach einem der Ansprüche 1 bis 11, **dadurch gekennzeichnet, dass** die zweiten Schieber (30) parallel zur Längsachse (31) Langlöcher (37) aufweisen und an der Seitenwand (16) mittels eines Zwischenringes (42) und einer Schraube (41) befestigte Führungselemente (38) als in den Langlöchem (37) aufnehmbare Lagerzapfen für die zweiten Schieber (30) dienen, wobei die Zwischenringe (42) im wesentlichen senkrecht auf den Seitenwänden (16) stehen und somit über die Seitenwände (16) vorragende Nocken bilden, welche beim Aufsetzen des Deckels (1) auf das oberste Instrumententablar (4) respektive beim Stapeln mehrerer Instrumententablare (4) in entsprechende, gegen die Unterseite (7) offene Öffnungen (45) an den Seitenwänden (16) in Eingriff bringbar sind.

13. Vorrichtung nach Anspruch 12, **dadurch gekennzeichnet, dass** die Zwischenringe (42) verschiedene Aussendurchmesser aufweisen, so dass durch eine komplementäre Ausgestaltung der Öffnungen (45) an den Seitenwänden (16) der Deckel (1) und die Instrumententablare (4) nur in einer bezüglich der Schmalseiten (27) respektive Längsseiten (25) definierten Ordnung gestapelt werden können und nicht um 180° verdreht stapelbar sind.

14. Vorrichtung nach Anspruch 12, **dadurch gekennzeichnet, dass** die Langlöcher (37) und die dazu korrespondierenden mittels eines Zwischenringes (42) und einer Schraube (41) befestigten Führungselemente (38) an jeder der beiden Schmalseiten (27) und bezüglich der Längsachse (31) axial, verschiedene Abstände aufweisen, so dass der Deckel (1) und die Instrumententablare (4) nur in einer bezüglich der Schmalseiten (27) respektive Längsseiten (25) definierten Ordnung gestapelt werden können und nicht um 180° verdreht stapelbar sind.

## Claims

1. A device for storing objects, in particular surgical instruments and/or implants, with
A) at least one shell-shaped instrument tray (4) comprising a base (9), an upper side (6) that is essentially parallel to the horizontal outline of the base (9), an underside (7), as well as lateral walls (16) that are provided on the base (9) essentially perpendicularly, while the at least one instrument tray (4) can be stacked with a further instrument tray(4); and
B) a lid (1) comprising a cover plate (17) having the same horizontal outline as the base (9), an upper side (14), an underside (13) and at least one displaceable locking mechanism (3) for the purpose of detachable fastening of the lid (1) on the upper side (6) of an instrument tray (4), whereby
C) each instrument tray (4) comprises locking means (10), and by displacing them each instrument tray (4) can be detachably fastened with an instrument tray adjacent below, **characterised in that**
D) the locking means (10) comprise upper locking elements (11) facing the upper side (6) and lower locking elements (12) facing the underside (7), and
E) the locking mechanism (3) also comprises lower locking elements (12) facing the lid underside (13), while
F) the lower locking elements (12) can be detachably engaged with the upper locking elements (11) of an instrument tray (4) adjacent below and the locking elements (11, 12) can be synchronously displaced by displacing the at least one locking mechanism (3) on the lid (1).

2. A device according to claim 1, **characterised in that** the lower locking elements (12) can be detachably engaged with the base (9) of an instrument tray (4) adjacent below by displacing the locking mechanism (3) parallel to the horizontal projection.

3. A device according to claim 1 or 2, **characterised in that** the at least one locking mechanism (3) comprises a handle (26) to displace the locking mechanism (3).

4. A device according to any one of claims 1 to 3, **characterised in that** the lateral walls (16) of an instrument tray *i* (4) has a height (Hᵢ), an internal side (2) and an external side (32) and are provided on the underside (7), and that the lateral walls (16) are outward cranked from the underside (7) to a depth of (Tᵢ), due to which a shoulder (20) is formed on the internal side (2).

5. A device according to any one of claims 1 to 4, **characterised in that** the horizontal projection of the cover plate (17) and of each base (9) is essentially rectangular and comprises two parallel longitudinal sides (25) and two narrow sides (27) which are also parallel, while two locking mechanisms (3) are provided on the lid (1) and two locking means (10) on an instrument tray (4), in each case on the narrow sides (27).

6. A device according to claim 4 or 5, **characterised in that** the depth (Tᵢ) of each instrument tray i (4) is so dimensioned, that the shoulder (20) can be rested on the base (9) of an instrument tray *i*+1 (4) adjacent below.

7. A device according to claim 4 or 5, **characterised in that** the depth (Tᵢ) of each instrument tray *i* (4) is so dimensioned, that when the instrument trays (4) are stacked an air gap (40) remains between the shoulders (20) on the lateral walls (16) and the bases (9) of the respective instrument tray adjacent below.

8. A device according to any one of claims 1 to 7, **characterised in that** the cover plate (17) and each base (9) are provided with ventilation holes (24) passing through them perpendicularly.

9. A device according to any one of claims 5 to 8, **characterised in that** each locking mechanism (3) comprises a first bar-shaped slide (29) and each locking means (10) comprises a second bar-shaped slide (30), while the slides (29, 30) comprise a longitudinal axis (31) that runs parallel to the narrow side (27) and parallel to the horizontal outline of the cover plate (17) or of the base (9).

10. A device according to claim 9, **characterised in that** the upper locking elements (11) are provided on the slides (30) as recesses which are perpendicular to the longitudinal axis (31) and the lower locking elements (12) are provided on the slides (29, 30) as corresponding protuberances, the base (9) comprises locking holes (21) passing through perpendicularly to the horizontal outline, through which the lower locking elements (12) can be passed, and that the lower locking elements (12) comprise a slot (9) extending parallel to the longitudinal axis (31), which slot can be engaged with the base (9) of the instrument tray (4) adjacent below by displacing the slide (29, 30) via the lower locking elements (12) guided through the locking holes (21) of an instrument tray (4) adjacent below.

11. A device according to any one of claims 4 to 10, **characterised in that** the base (9) of an instrument tray *i* (4) comprises *n* cavities (5) on the upper side (6), the shape of each cavity (5) defining the surgical instrument and/or implant intended for storage, the cavity *j* (5) has a depth of Tⱼ and a base area Gⱼ (8), and that the dimensions H*i* and T*i* of the upper instrument tray (4) of two instrument trays (4) stacked on top of one another and the depths Tⱼ of the lower and upper instrument tray (4) are so determined, that the surgical instruments or implants placed into the cavities (5) of the lower instrument tray (4) in a determined manner can be secured perpendicularly to the base (9).

12. A device according to any one of claims 1 to 11, **characterised in that** the second slides (30) have slots (37) that are parallel to the longitudinal axis (31) and guide elements (38) fastened on the lateral walls (16) by means of an intermediate ring (42) and of a screw (41) serve as mounting spigots for the second slide (30) which can be accommodated in the slots (37), while the intermediate rings (42) are essentially perpendicular on the lateral walls (16) and consequently form lugs protruding past the lateral walls (16), which lugs can be engaged by the openings (45) on the lateral walls (16) open to the underside (7) when the lid (1) is placed on the uppermost instrument tray (4) or when stacking a plurality of instrument trays (4).

13. A device according to claim 12, **characterised in that** the intermediate rings (42) have various outside diameters, so that by a complementary construction of the openings (45) on the lateral walls (16) the lid (1) and the instrument tray (4) can be stacked only in a manner defined by the narrow sides (27) or the longitudinal sides (25) and cannot be stacked rotated about 180°.

14. A device according to claim 12, **characterised in that** the slots (37) and the corresponding guide elements (38) fastened by means of an intermediate ring (42) and of a screw (41) are at various axial distances from one another on each of the two narrow sides (27) and relative the longitudinal axis (31), so that the lid (1) and the instrument tray (4) can be stacked only in a manner defined by the narrow sides (27) or the longitudinal sides (25) and cannot be stacked rotated about 180°.

## Revendications

1. Dispositif permettant de conserver des objets, notamment des instruments et/ou des implants chirurgicaux, comprenant
A) au moins une tablette à instruments (4) en forme de coque, laquelle comprend un fond (9), une face supérieure (6) essentiellement parallèle au plan horizontal du fond (9), une face inférieure (7) ainsi que des parois latérales (16) fixées au fond (9) et ce essentiellement de manière perpendiculaire à celui-ci, la tablette à instruments (4) - dont il existe au moins une - pouvant être empilée sur une autre tablette à instruments (4); et
B) un couvercle (1) qui comprend une plaque de recouvrement (17) dont le plan horizontal est identique à celui du fond (9), une face supérieure de couvercle (14), une face inférieure de couvercle (13) et au moins un mécanisme de fermeture (3) pouvant être actionné et permettant de fixer, de manière amovible, le couvercle (1) sur la face supérieure (6) d'une tablette à instruments (4),
C) chaque tablette à instruments (4) comprenant des moyens de verrouillage (10) dont le déplacement permet de fixer, de manière amovible, chaque tablette à instruments (4) à une tablette à instruments (4) avoisinante située juste au-dessous,
**caractérisé en ce que**
D) les moyens de verrouillage (10) comprennent des éléments de verrouillage supérieurs (11) dirigés vers la face supérieure (6) et des éléments de verrouillage inférieurs (12) dirigés vers la face inférieure (7); et
E) le mécanisme de fermeture (3) comprend également des éléments de verrouillage inférieurs (12) dirigés vers la face inférieure de couvercle (13),
F) les éléments de verrouillage inférieurs (12) pouvant être enclenchés, de manière amovible, dans les éléments de verrouillage supérieurs (11) d'une tablette à instruments (4) avoisinante située juste au-dessous et les éléments de verrouillage (11;12) pouvant être déplacés de manière synchronisée par actionnement du mécanisme de fermeture (3) - dont il existe au moins un - situé sur le couvercle (1).

2. Dispositif selon la revendication 1, **caractérisé en ce que** les éléments de verrouillage inférieurs (12) peuvent, par actionnement du mécanisme de fermeture (3) parallèlement au plan horizontal, être enclenchés de manière amovible dans le fond (9) d'une tablette à instruments (4) avoisinante située juste au-dessous.

3. Dispositif selon la revendication 1 ou 2, **caractérisé en ce que** le mécanisme de fermeture (3) - dont il existe au moins un - comprend une manette (26) permettant d'actionner le mécanisme de fermeture (3).

4. Dispositif selon l'une des revendications 1 à 3, **caractérisé en ce que** les parois latérales (16) d'une tablette à instruments i(4) présentent une hauteur (Hᵢ), une face intérieure (2) et une face extérieure (32) et sont fixées sur la face inférieure (7), et **en ce que** les parois latérales (16) sont coudées vers l'extérieur depuis la face inférieure (7) jusqu'à une profondeur (Tᵢ), de manière à former un épaulement (20).

5. Dispositif selon l'une des revendications 1 à 4, **caractérisé en ce que** le plan horizontal de la plaque de recouvrement (17) et de chaque fond (9) est essentiellement rectangulaire et comprend deux côtés longitudinaux (25) parallèles et deux côtés étroits (27) également parallèles, deux mécanismes de fermeture (3) étant disposés sur le couvercle (1) et deux moyens de verrouillage (10) étant disposés sur une tablette à instruments (4), et ce respectivement sur les côtés étroits (27).

6. Dispositif selon la revendication 4 ou 5, **caractérisé en ce que** la profondeur (Tᵢ) de chaque tablette à instruments i(4) est dimensionnée de telle sorte que l'épaulement (20) peut être disposé de manière à reposer sur le fond (9) d'une tablette à instruments i+1 (4) avoisinante située juste au-dessous.

7. Dispositif selon la revendication 4 ou 5, **caractérisé en ce que** la profondeur (Tᵢ) de chaque tablette à instruments i(4) est dimensionnée de telle sorte que, lorsque les tablettes à instruments (4) sont empilées les unes sur les autres, il subsiste un espace d'air (40) entre les épaulements (20) formés au niveau des parois latérales (16) et les fonds (9) des tablettes à instruments (4) avoisinantes situées respectivement juste au-dessous.

8. Dispositif selon l'une des revendications 1 à 7, **caractérisé en ce que** la plaque de recouvrement (17) et chaque fond (9) sont pourvus de trous de ventilation (24) qui les traversent essentiellement de manière perpendiculaire.

9. Dispositif selon l'une des revendications 5 à 8, **caractérisé en ce que** chaque mécanisme de fermeture (3) comprend un premier coulisseau (29) en forme de tige et chaque moyen de verrouillage (10) un deuxième coulisseau (30) en forme de tige, les coulisseaux (29;30) comprenant un axe longitudinal (31) s'étendant parallèlement au côté étroit (27) et parallèlement au plan horizontal de la plaque de recouvrement (17) ou du fond (9), et **en ce que** les coulisseaux (29;30) peuvent coulisser parallèlement audit axe longitudinal (31).

10. Dispositif selon la revendication 9, **caractérisé en ce que** les éléments de verrouillage supérieurs (11) sont réalisés sur les coulisseaux (30) sous forme de creux ménagés perpendiculairement à l'axe longitudinal (31) et les éléments de verrouillage inférieurs (12) sont réalisés sur les coulisseaux (29;30) sous forme de saillies correspondantes, **en ce que** le fond (9) comprend des trous de verrouillage (21) traversant perpendiculairement le plan horizontal, à travers lesquels peuvent être introduits les éléments de verrouillage inférieurs (12), et **en ce que** les éléments de verrouillage inférieurs (12) comprennent une fente (19) s'étendant parallèlement à l'axe longitudinal (31) qui peut, lorsque les éléments de verrouillage inférieurs (12) sont introduits dans les trous de verrouillage (21) d'une tablette à instruments (4) avoisinante située juste au-dessous, entrer en prise avec le fond (9) de ladite tablette à instruments (4) avoisinante située juste au-dessous, et ce par déplacement du coulisseau (29;30).

11. Dispositif selon l'une des revendications 4 à 10, **caractérisé en ce que** le fond (9) d'une tablette à instruments i(4) comprend, sur la face supérieure (6), n creux (5) dont la forme détermine l'instrument et/ou l'implant chirurgical destiné à être conservé dans chaque creux (5), **en ce que** le creux j(5) présente une profondeur Tⱼ et une surface de base Gⱼ(8), et **en ce que** les dimensions Hⱼ et Tⱼ de la tablette à instruments (4) supérieure de deux tablettes à instruments (4) devant être empilées l'une sur l'autre concordent avec les profondeurs Tⱼ de la tablette à instruments (4) inférieure et de la tablette à instruments (4) supérieure de telle sorte que les instruments et/ou les implants chirurgicaux placés dans l'ordre prédéfini dans les creux (5) de la tablette à instruments (4) inférieure puissent être fixés perpendiculairement au fond (9) par les surfaces de base (8) de la tablette à instruments (4) supérieure.

12. Dispositif selon l'une des revendications 1 à 11, **caractérisé en ce que** les deuxièmes coulisseaux (30) présentent des trous oblongs (37) s'étendant parallèlement à l'axe longitudinal (31) et **en ce que** des éléments de guidage (38) fixés sur la paroi latérale (16) au moyen d'un anneau intermédiaire (42) et d'une vis (41) servent aux deuxièmes coulisseaux (30) de tourillons pouvant être logés dans les trous oblongs (37), les anneaux intermédiaire (42) s'étendant essentiellement de manière perpendiculaire aux parois latérales (16) et formant ainsi des cames faisant saillie par rapport aux parois latérales (16) qui peuvent être enclenchées, lors de la mise en place du couvercle (1) sur la tablette à instruments (4) située tout en haut ou lors de l'empilage de plusieurs tablettes à instruments (4), dans des ouvertures correspondantes (45) ménagées dans les parois latérales (16) et ouvertes vers la face inférieure (7).

13. Dispositif selon la revendication 12, **caractérisé en ce que** les anneaux intermédiaires (42) présentent différents diamètres extérieurs, de sorte que, grâce à une forme complémentaire des ouvertures (45) ménagées dans les parois latérales (16), le couvercle (1) et les tablettes à instruments (4) ne puissent être empilés que dans un ordre défini en ce qui concerne les côtés étroits (27) ou les côtés longitudinaux (25) et qu'il soit impossible de les empiler à l'envers, en leur faisant effectuer un pivotement de 180°.

14. Dispositif selon la revendication 12, **caractérisé en ce que** les trous oblongs (37) et les éléments de guidage (38) correspondants fixés au moyen d'un anneau intermédiaire (42) et d'une vis (41) présentent des écarts différents sur chacun des deux côtés étroits (27) et par rapport à l'axe longitudinal (31), de sorte que le couvercle (1) et les tablettes à instruments (4) ne puissent être empilés que dans un ordre défini en ce qui concerne les côtés étroits (27) ou les côtés longitudinaux (25) et qu'il soit impossible de les empiler à l'envers en leur faisant effectuer un pivotement de 180°.
